Europäisches Patentamt

(19) European Patent Office

Office européen des brevets

(11) Publication number: **0 030 953**
**B1**

(12) **EUROPEAN PATENT SPECIFICATION**

(45) Date of publication of patent specification: **26.09.84**

(21) Application number: **80901197.6**

(22) Date of filing: **13.06.80**

(86) International application number:
**PCT/SE80/00170**

(87) International publication number:
**WO 80/02801 24.12.80 Gazette 80/29**

(51) Int. Cl.³: **A 61 N 1/04, A 61 N 1/36**

(54) **ENDOCARDIAL, IMPLANTABLE LEAD FOR PACEMAKER.**

(30) Priority: **14.06.79 SE 7905227**

(43) Date of publication of application:
**01.07.81 Bulletin 81/26**

(45) Publication of the grant of the patent:
**26.09.84 Bulletin 84/39**

(84) Designated Contracting States:
**FR**

(56) References cited:
**US-A-3 416 533**
**US-A-3 890 977**
**US-A-3 939 843**
**US-A-4 057 067**
**US-A-4 154 247**

(73) Proprietor: **REENSTIERNA, Bertil**
**Borgeby 11**
**S-230 50 Bjärred (SE)**

(72) Inventor: **REENSTIERNA, Bertil**
**Borgeby 11**
**S-230 50 Bjärred (SE)**

(74) Representative: **ROSTOVANYI, Peter et al**
**AWAPATENT AB Box 5117**
**S-200 71 Malmö (SE)**

Courier Press, Leamington Spa, England.

## Description

This invention relates to an endocardial, implantable lead of the type indicated in the preamble of claim 1.

Many leads of this kind have become known for transferring stimulating pulses from a pacemaker box to the heart or for supplying the heart's own pulses to the pacemaker box.

To establish reliable contact between the auricle electrode and the cardiac tissue there have been developed a great many means provided on or in the lead and being of the type of hooks, barbs and screws. These means are adapted to be actuated from outside the patient's body so as to penetrate into the cardiac tissue and carry along the electrode, thereby establishing contact with the cardiac tissue.

The disadvantages associated with such means are that they can easily damage the cardiac tissue and can also easily come loose so that the requisite reliable contact with the cardiac tissue is jeopardized.

US A 4,154,247 and 3,890,977 show curved portions of a lead having electrodes to contact the cardiac tissue. The curved portion may consist of a helical loop the general plane of which includes the lead portions connected to the loop. These types of lead portions act like a leaf spring under the compressions and expansions of the heart, thereby creating a risk of injuring the heart inner wall, especially at the tip portion of the lead in contact with the heart ventrical."

The object of the invention is to provide an endocardial, implantable lead of this type, which without the aid of tissue-tearing means can establish good contact between the auricle electrode and the cardiac tissue.

This object is realized in accordance with the present invention by means of a lead of the type described in the foregoing and possessing the characteristic features that appear from the appended claims.

The invention will be described in greater detail below with reference to the accompanying drawings.

Fig. 1 is a longitudinal section of a human heart with a lead according to the invention implanted therein,

Fig. 2 is a section on line II—II in Fig. 1, and

Fig. 3 is an enlarged view of a portion (III in Fig. 1) of the lead.

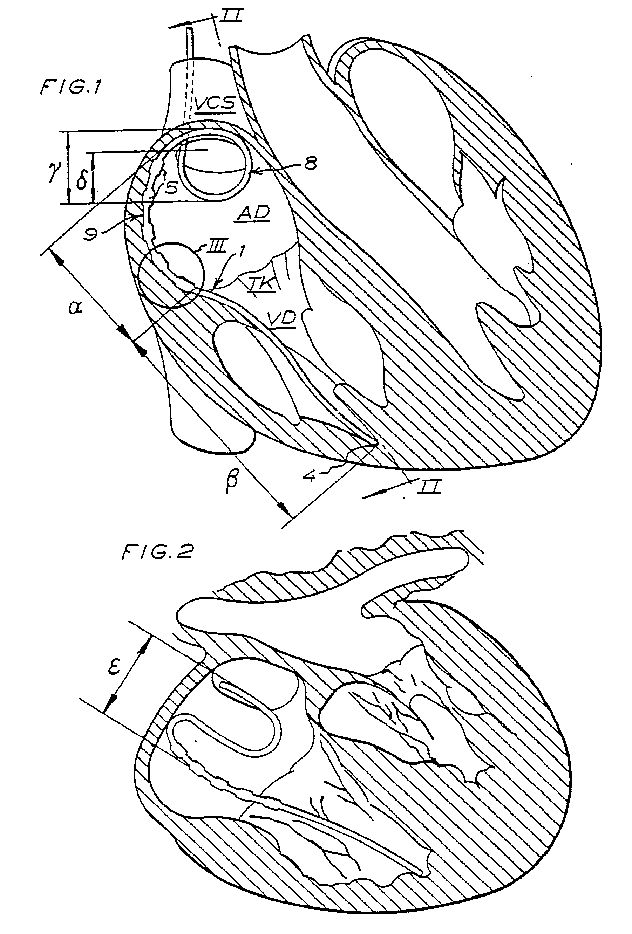

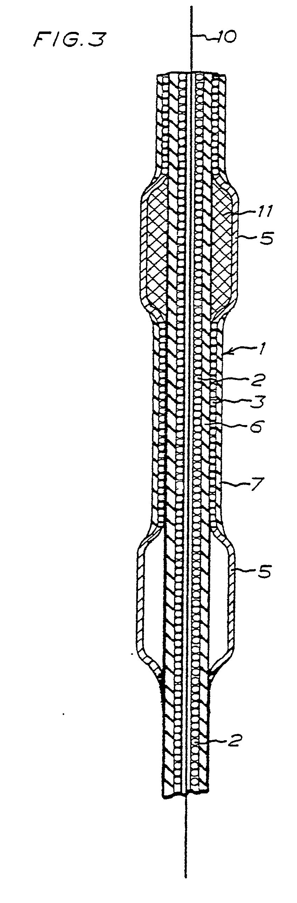

Fig. 1 shows an intravascular endocardial lead 1 which is implanted in a human heart via a punctured body vessel (not shown), such as one jugular vein, and via the superior vena cava VCS (vena cava superior). It is now referred to Fig. 3 where a portion III in Fig. 1 of the lead is shown in enlargement. The lead 1 comprises two flexible helical concentrically arranged conductors 2 and 3 of some metal or metal alloy conventional for the purpose contemplated, such as a platinum/iridium alloy. At its distal end the inner conductor 2 is connected to an electrode 4 in contact with the apex of the right heart ventricle VD (ventriculus dexter). At and adjacent to its distal end which is upstream of the distal end of the inner conductor 2, the outer conductor 3 is connected to auricle electrodes 5 which are spaced apart in the longitudinal direction of the lead 1 and are in contact with the inner wall, the endocardium, of the right auricle AD (atrium dexter) to receive heart pulses.

At their proximal ends the conductors 2, 3 are connectible to an implantable heart stimulating circuit adapted, in response to the heart pulses received from the electrodes 5, to generate heart stimulating pulses and transfer these generated heart stimulating pulses via the electrode 4 to the right ventricle VD. The lead 1 illustrated is thus adapted for auricle controlled heart stimulation of a human heart with partial or total AV-block.

Sheaths 6, 7 are formed about the conductors 2, 3. The inner sheath 6 embraces the inner conductor 2 from the proximal end thereof to the electrode 4 and separates said inner conductor from the outer conductor 3. The outer sheath 7 consists of a continuous portion extending from the proximal end of the outer conductor 3 to the first auricle electrode 5, as viewed from said proximal end, and of several spaced apart successive portions embracing the outer conductor 3, between which portions the other auricle electrode 5 protrudes. The last-mentioned electrodes are in the form of rings which are electrically connected to the outer conductor 3, for example by soldering, at their upper and lower edges and are made from a tissue-compatible material, such as the same platinum/iridium alloy as the conductors 2, 3.

The sheaths 6, 7 are made from a tissue-compatible electrically insulating elastomer, such as a silicon rubber. The inner sheath 6, when being moulded into a tube over that part of its length which is to lie in the auricle AD, has been given the shape of a loop or a one-turn coil 8 and a curved portion 9 connecting onto said coil and containing the auricle electrodes 5. The coil 8 can be a right-hand turn, as illustrated, or a left-hand turn, as seen towards the distal end of the lead 1. The coil is open, i.e. the intersection of the lead 1 with itself takes place on two levels, as will appear from Fig. 2, and the diameter or largest cross-section of the coil is adapted to prevent the portion of the lead 1 downstream of the coil to move outwards through the vena cava VCS. The coil 8 extends in the longitudinal direction of the lead 1, such that the line of intersection between the major plane of the coil and longitudinal planes of the lead 1 makes an angle with the longitudinal direction of the lead 1, which angle can be about 45°. The curved portion gently joins the coil 8 so that its centre of curvature lies on the same side of the lead 1 as the centre of curvature of the immediately connecting loop portion. The curvature of the portion 9 is such that by the intermediary of at least one auricle electrode said portion will be sure to contact, and be slightly urged against, the endocardium in the auricle AD, preferably following the general curvature of the endocardium, over a considerable distance $\alpha$, approximately from the mouth of the vena cava VCS in the auricle AD to the tricuspid valve TK, as will appear from Fig. 1, which is realised by a certain overdimensioning of the curved portion 9, such that the inherent resilience of the lead 1 realizes the above-mentioned urging.

The inner sheath 6 can be provided with the portions 8 and 9 by extrusion of a tube in a straight state, whereupon this tube before vulcanization is placed in a jig which is provided with release agent or passed onto a wire template of the same shape as that desired (see Fig. 1) for the lead portions 8, 9. The tube is then vulcanized in the jig or on the template. After insertion of the inner conductor 2 into the resulting tube which has been given the desired shape and which has optionally been expanded with the aid of gas to permit such insertion, and after passing of the tube onto the conductor 3 with the annular electrodes 5 attached thereto, the outer sheath 7 can be moulded over the outer conductor 3 between and beyond the electrodes 5.

After accomplished vulcanization the lead 1 is resiliently biased to the shape of the coil turn 8 and the curved portion 9, and after straightening the lead 1 can resume said desired curvature which is illustrated in Figs. 1 and 2 and has a coil 8 and curved portion 9. The lead portions on both sides of these members 8, 9 are unbiased.

Straightening of the lead 1 has to be carried out before and during insertion of the electrodes 4 and 5 in the heart. To this end, a relative stiff metal wire 10 is introduced into the central opening in the lead 1 defined by the conductor 2 and is moved all the way up to the electrode 4. Said wire simultaneously serves as straightening device for the loop or coil 8 and for the curved portion 9 and as a guide means for the ventricle electrode 4 for guiding it to the heart apex. After said electrode 4 has been brought into correct position, the lead being paid out simultaneously as the wire 10 is withdrawn through the puncture in the body vessel, for example one jugular vein, the wire 10 can be entirely withdrawn through said puncture. By reason of its spring bias described, the lead 1 is brought into the shape shown in Figs. 1 and 2. With this shape of the lead 1, the auricle electrodes 5 positively bear against the endocardium of the auricle also when the heart is working, and the coil 8 prevents the part of the lead 1 located in the heart from moving out through the vena cava VCS, and results in the positive abutment of the electrode tip against the lower apex of the ventricle VD.

Thus, it will be appreciated that the coil 8 serves as a member absorbing the movements of the heart, and not transferring these movements to the other portions of the lead 1.

Being spring biased, the lead in the heart can take part in the movement of the heart simultaneously as the electrodes 4 and 5 remain in contact with the heart at the points illustrated in Fig. 1. The loop or coil 8 unloads the lead 1 from the stresses caused by the heart movements.

An annular magnet 11 can, if desired, be arranged in the annular space radially inside each ring electrode 5 or a number of said ring electrodes 5, as is illustrated in Fig. 3. Said magnet 11 can be caused to cooperate with a magnet placed on the outer side of the patient's body for fixation of the electrodes 5 to the myocardium until fibrin is precipitated onto the lead.

In a similar manner the lead 1 may contain a magnet also in the vicinity of its distal end to maintain the electrode 4 in contact with the myocardium in the ventricle VD until fibrin is precipitated, all in conformity with my U.S. Patent No. 4,162,679.

The illustrated plurality of auricle electrodes is not necessary. It may very well be replaced by a few or even a single auricle electrode 5 in the curved portion 9. In that case, the electrode 5 is located at such as point of said portion that a positive contact is obtained with the endocardium in the right auricle AD.

The dimensions of the loop or coil 8 and the curved portion 9 are preferably determined on a model of the patient's heart. The following dimensional statements may, however, serve as a guide where the heart of an adult is concerned. Length $\alpha$ of curved portion $9 = 32$ mm, length $\beta$ from the distal end of the curved portion 9 approximately at the tricuspid TK to the distal end of the lead $= 85$ mm, diameter $\gamma$ of the approximately circular coil $8 = 22$ mm, distance $\delta$ between the mouth of the vena cava VCS in the auricle AD and the lowermost portion of the coil $= 15$ mm, distance of intersection $\varepsilon$ at the coil $= 25$ mm, and the total length of portions 8 and $9 = 130$ mm.

An alternative embodiment (not shown) of the lead 1 is a strip lead where the helical conductors 2 and 3 are placed in juxtaposition and each conductor is embraced by a sheath 6 and 7, with the exception that the conductor 3 comprises the auricle electrodes 5 which are devoid of sheaths. The conductors 2 and 3 may be enclosed together with the sheaths 6, 7 by a common catheter cover of a more rigid material than the sheaths 6 and 7 and movable in its longitudinal direction along the sheaths 6 and 7. The conductor 2 may be provided with a guide wire for guiding the electrode 4 into the right ventricle VD, and the sheath 7 for the conductor 3 is spring-biased, as described in the foregoing. The catheter cover serves the same straightening function as the wire 10 in the embodiment illustrated and can, after implantation in the heart, be withdrawn to restore the slackened shape of the sheath and the conductor 3.

Instead of either sheath 6, 7 or the two sheaths 6 and 7 the electrical conductor or conductors 2, 3 can be biased to the desired shape (illustrated in Fig. 1) of the coil 8 and the curved portion 9, which helical conductors in the produced straight state are placed in a conventional manner, in a jig of the desired configuration for the lead 1, and heated, whereafter they are allowed to cool for obtaining memory. A lead 1 having an electrical conductor and of coil and/or loop form for contact with the auricle by means of auricle electrodes can of course be utilized without the aid of a ventricle electrode for solely auricle stimulation.

## Claims

1. Endocardial, implantable lead for connection with an implantable pacemaker, said lead (1) comprising an electrical conductor (3) adapted, via at least one electrode (5) connected thereto and in contact with the inner wall of a heart auricle (AD), to transfer stimulating pulses to the heart or collect heart pulses from the heart, the lead (1) being spring-biased along a predetermined portion (8, 9) of its length to assume a curved shape, said curvature being dimensioned so as to bear against the inner wall of the heart auricle along a substantial length ($\alpha$) of said curvature, which contains said electrode or electrodes (5), characterised in that the predetermined portion (8, 9) further includes an open helical coil for absorbing forces exerted on the coil by movement of the heart and being connected to the substantial length ($\alpha$) of curvature, the axis of the coil not being perpendicular to the general direction of the lead on the proximal side of the coil.

2. A lead as claimed in claim 1, characterised in that the bias is generated by one or more tubular members (6, 7) of an elastomer, such as silicon rubber, which embraces and/or is embraced by the conductor (3) but leaves said electrode or electrodes (5) free for contact with the inner wall of the auricle (AD).

3. A lead as claimed in claim 1 or 2, characterised in that a relatively rigid elongated means (10) is adapted, in releasable positive connection with said predetermined portion, to counteract the bis forces and permit insertion of said portion in the heart in a substantially straightened state.

4. A lead as claimed in any one of claims 1—3, characterised in that the shape of said substantial length ($\alpha$) of the curvature (8, 9) generally corresponds to the curvature of the inner wall of the auricle (AD) in a longitudinal section thereof, from approximately the mouth of the superior vena cava (VCS) in the auricle to the tricuspid valves (TK).

5. A lead as claimed in any one of claims 1—4, characterised in that the open helical coil (8) is of slightly larger transverse dimension than the transverse dimension of the mouth of the superior vena cava (VCS) in the auricle (AD) and gently connects onto said substantial length ($\alpha$) of curvature.

6. A lead as claimed in any one of claims 1—4, characterised in that the angle between axis of the open helical coil (8) and the general direction of the lead (1) on the proximal side of the coil (8) is about 45°.

7. A lead as claimed in any one of claims 1—6, characterised in that the electrodes (5) consist of annular elements spaced apart along said substantial length ($\alpha$) of curvature.

8. A lead as claimed in any one of claims 3—7, characterised in that the conductor (3) is helically wound and that the relatively rigid elongated means (10) is a metal wire which is slidably arranged in the opening of the helically wound conductor (3).

9. A lead as claimed in any one of claims 3—8, characterised in that it contains a second electrical conductor (2) which extends past the first conductor by a length ($\beta$) which contains a second electrode (4) arranged to contact the inner wall of a heart ventricle (VD).

10. A lead as claimed in claim 9, characterised in that the conductors (2, 3) are coaxial and insulated by said tubular members (6, 7), the auricle electrode or electrodes (5) being connected to the outer conductor (3).

## Patentansprüche

1. Endokardiale, implantierbare Leitung zum Anschluss an einen implantierbaren Herzschrittmacher, welche Leitung (1) einen elektrischen Leiter (3) umfasst, der über zumindest eine daran angechlossene und mit der Innenwand eines Herzvorhofs (AD) in Berührung stehende Elektrode (5) Reizimpulse an das Herz überträgt oder Herzimpulse vom Herz ableitet, wobei die Leitung (1) längs eines vorbestimmten Teils (8, 9) ihrer Länge unter Federvorspannung steht, um eine gekrümmte Form anzunehmen, und wobei diese Krümmung derart bemessen ist, dass sie über einen wesentlichen, die genannte Elektrode oder Elektroden (5) umfassenden Teil ($\alpha$) ihrer Länge gegen die Innenwand des Herzvorhofs anliegt, dadurch gekennzeichnet, dass der vorbestimmte Teil (8, 9) ausserdem eine offene, schraubenlinienförmige Schlaufe umfasst, die zur Aufnahme der von den Herzbewegungen auf die Schlaufe ausgeübten Kräfte dient und an den wesentlichen Teil ($\alpha$) der Krümmung angeschlossen ist, wobei die Achse der Schlaufe zu der allgemeinen Richtung der Leitung auf der proximalen Seite der Schlaufe nicht senkrecht ist.

2. Leitung nach Anspruch 1, dadurch gekennzeichnet, dass die Vorspannung durch einen oder mehrere, schlauchförmige Teile (6, 7) aus einem Elastomer, beispielsweise Silikongummi, erzeugt ist, die den Leiter (3) umschliessen und/oder von ihm umschlossen sind, jedoch die genannte Elektrode oder Elektroden (5) zur Berührung mit der Innenwand des Vorhofs (AD) frei lassen.

3. Leitung nach Anspruch 1 oder 2, dadurch gekennzeichnet, dass ein verhältnismässig steifes, längliches Glied (10) vorgesehen ist, das in lösbarer Kraftverbindung mit dem genannten vorbestimmten Teil den Vorspannungskräften entgegenwirkt und das Einführen des Teils ins Herz in hauptsächlich ausgerichtetem Zustand ermöglicht.

4. Leitung nach einem der Ansprüche 1—3, dadurch gekennzeichnet, dass die Form des genannten wesentlichen Teils (α) der Krümmung (8, 9) allgemein der Krümmung der Innenwand des Herzvorhofs (AD) im Längsschnitt davon entspricht, etwa von der Mündung der oberen Hohlvene (VCS) in den Herzvorhof bis zu den Trikuspidalklappen (TK).

5. Leitung nach einem der Ansprüche 1—4, dadurch gekennzeichnet, dass die offene, schraubenlinienförmige Schlaufe (8) eine Querabmessung hat, die etwas grösser ist als die Querabmessung der Mündung der oberen Hohlvene (VCS) in den Herzvorhof (AD), und sich sanft an den wesentlichen Teil (α) der Krümmung anschliesst.

6. Leitung nach einem der Ansprüche 1—4, dadurch gekennzeichnet, dass der Winkel zwischen der Achse der offenen, schraubenlinienförmigen Schlaufe (8) und der allgemeinen Richtung der Leitung (1) auf der proximalen Seite der Schlaufe (8) etwa 45° beträgt.

7. Leitung nach einem der Ansprüche 1—6, dadurch gekennzeichnet, dass die Elektroden (5) aus ringförmigen, in gegenseitigem Abstand längs des genannten, wesentlichen Krümmungsteils (α) angeordneten Elementen bestehen.

8. Leitung nach einem der Ansprüche 3—7, dadurch gekennzeichnet, dass der Leiter (3) schraubenlinienförmig gewickelt ist, und dass das verhältnismässig steife, längliche Glied (10) ein Metalldraht ist, der in der Öffnung des schraubenlinienförmig gewickelten Leiters (3) gleitbar angeordnet ist.

9. Leitung nach einem der Ansprüche 3—8, dadurch gekennzeichnet, dass sie einen zweiten elektrischen Leiter (2) umfasst, der sich mit einem Teil (β), welcher eine zweite, zur Berührung mit der Innenwand einer Herzkammer (VD) angeordnete Elektrode (4) enthält, am erstgenannten Leiter vorbeierstreckt.

10. Leitung nach Anspruch 9, dadurch gekennzeichnet, dass die Leiter (2, 3) koaxial und mittels der genannten schlauchförmigen Teile (6, 7) isoliert sind, wobei die Vorhofelektrode oder -elektroden (5) an den äusseren Leiter (3) angeschlossen sind.

**Revendications**

1. Fil conducteur endocardiaque implantable destiné à être connecté à un stimulateur cardiaque implantable, le fil conducteur (1) comprenant un conducteur électrique (3) qui, par l'intermédiaire d'au moins une électrode (5) qui lui est reliée et qui est au contact de la paroi interne d'une oreillette du coeur (AD), est destiné à transférer des impulsions de stimulation au coeur à collecter des impulsions cardiaques du coeur, le fil conducteur (1) étant rappelé élastiquement le long d'une partie prédéterminée (8, 9) de sa longueur afin qu'il prenne une forme courbe, la courbure ayant des dimensions telles que le fil est en appui contre la paroi interne de l'oreillette du coeur sur une longueur importante (α) de ladite courbure, comprenant l'électrode ou les électrodes (5), caractérisé en ce que la partie prédéterminée (8, 9) comporte en outre une spire hélicoïdale ouverte destinée à absorber les forces exercées sur la spire par les déplacements du coeur et raccordée sur le tronçon de longueur notable (α) de la courbure, l'axe de la spire n'étant pas perpendiculaire à la direction générale du fil conducteur du côté proche de la spire.

2. Fil conducteur selon la revendication 1, caractérisé en ce que le rappel élastique est créé par un ou plusieurs organes tubulaires (6, 7) formés d'un élastomère tel qu'un caoutchouc de silicone, qui entoure le conducteur (3) et/ou est entouré par celui-ci, mais laisse l'électrode ou les électrodes (5) libres d'être au contact avec la paroi interne de l'oreillette (AD).

3. Fil conducteur selon l'une des revendications 1 et 2, caractérisé en ce que le dispositif allongé relativement rigide (10) est destiné à contrecarrer les forces de rappel et à permettre l'introduction de ladite partie dans le coeur à un état sensiblement rectiligne, lors d'un raccordement positif mais temporaire avec ladite partie prédéterminée.

4. Fil conducteur selon l'une quelconque des revendications 1 à 3, caractérisé en ce que la configuration du tronçon de longueur notable (α) de la courbure (8, 9) correspond de façon générale à la courbure de la paroi interne de l'oreillette (AD) dans une coupe longitudinale, de l'embouchure approximative de la veine cave supérieure (VCS) dans l'oreillette aux valves tricuspides (TK).

5. Fil conducteur selon l'une quelconque des revendications 1 à 4, caractérisé en ce que la spire hélicoïdale ouverte (8) a une dimension transversale légèrement supérieure à la dimension transversale de l'embouchure de la veine cave supérieure (VCS) dans l'oreillette (AD) et se raccorde progressivement au tronçon de longueur notable (α) de courbure.

6. Fil conducteur selon l'une quelconque des revendications 1 à 4, caractérisé en ce que l'angle formé par l'axe de la spire hélicoïdale ouverte (8) et la direction générale du fil conducteur (1) du côté proche de la spire (8) est d'environ 45°.

7. Fil selon l'une quelconque des revendications 1 à 6, caractérisé en ce que les électrodes (5) sont formées d'éléments annulaires espacés le long dudit tronçon de longueur notable (α) de courbure.

8. Fil conducteur selon l'une quelconque des revendications 3 à 7, caractérisé en ce que le conducteur (3) est enroulé en hélice et en ce que le dispositif alongé relativement rigide (10) est un fil métallique qui peut glisser dans l'orifice du conducteur enroulée en hélice (3).

9. Fil conducteur selon l'une quelconque des revendications 3 à 8, caractérisé en ce qu'il contient un second conducteur électrique (2) qui dépasse du premier conducteur d'une longueur (β) qui comprend une seconde électrode (4) destinée à être au contact de la paroi interne d'un ventricule cardiaque (VD).

10. Fil conducteur selon la revendication 9, caractérisé en ce que les conducteurs (2, 3) sont coaxiaux et isolés par des organes tubulaires (6, 7) l'électrode ou les électrodes auriculaires (5) étant connectées au conducteur externe (3).

0 030 953

FIG.1

FIG.2

1

FIG.3